(19) Europäisches Patentamt
European Patent Office
Office européen des brevets

(11) Numéro de publication : **0 682 028 A1**

(12) # DEMANDE DE BREVET EUROPEEN

(21) Numéro de dépôt : **95401015.3**

(22) Date de dépôt : **03.05.95**

(51) Int. Cl.[6] : **C07D 495/04,** C07F 5/02, A61K 31/415, // (C07D495/04, 333:00, 209:00)

(30) Priorité : **10.05.94 FR 9405716**

(43) Date de publication de la demande : **15.11.95 Bulletin 95/46**

(84) Etats contractants désignés : **AT BE CH DE DK ES FR GB GR IE IT LI LU NL PT SE**

(71) Demandeur : **SYNTHELABO**
**22, Avenue Galilée**
**F-92350 Le Plessis Robinson (FR)**

(72) Inventeur : **Bedoya Zurita, Manuel**
**Pintor Murillo 13,3 A**
**E-28110 Alcobendas, Madrid (ES)**
Inventeur : **Diaz Martin, Juan Antonio**
**Anastro 23, 1 -C**
**E-28033 Madrid (ES)**
Inventeur : **Del Sol Moreno, Gregorio**
**Candido Mateos 18 Portal 1,**
**Esc. 1, 6 -A**
**E-28035 Madrid (ES)**
Inventeur : **Martin Escudero Perez, Ulpiano**
**Alburquerque 15, 6 -D**
**E-28010 Madrid (ES)**
Inventeur : **Jimenez Bargueno, Maria Dolores**
**Paseo de la Chopera n 102, 4 B**
**E-28100 Alcobendas, Madrid (ES)**
Inventeur : **Romanach Ferrer, Magali**
**Vesubiana 22,**
**Getafe-Sector 3**
**E-28905 Madrid (ES)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés de 5,6-dihydro-4H-thiéno 3,4-c pyrrole, leur préparation et leur application en thérapeutique.**

(57) Dérivés de 5,6-dihydro-4*H*-thiéno[3,4-c)pyrrole de formule générale (I)

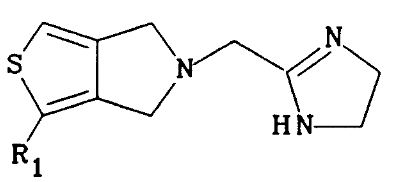

(I)

dans laquelle $R_1$ représente soit un groupe cyano, $C_{1-4}$ alcoxy, $C_{1-4}$ alkylthio, $C_{1-4}$ alkylsulfonyle, pyridyle, 3,4-diméthoxyphényle ou cyclopropyle éventuellement substitué par un ou deux groupes alkyle, soit un groupe COR où R est un groupe $C_{1-4}$ alkyle, phényle ou pipéridinyle, soit un groupe benzyle substitué par un ou plusieurs atomes d'halogène et/ou groupes $C_{1-4}$ alkyle linéaire ou ramifié, $C_{1-4}$ alcoxy linéaire ou ramifié ou $CO_2R'$ où R' est un groupe $C_{1-4}$ alkyle linéaire ou ramifié, soit un groupe naphtylméthyle, ainsi que leurs sels d'addition à des acides pharmaceutiquement acceptables.

EP 0 682 028 A1

La présente invention a pour objet des dérivés de 5,6-dihydro-4*H*-thiéno[3, 4-*c*]pyrrole, leur préparation et leur application en thérapeutique.

Les composés de l'invention répondent à la formule générale (I)

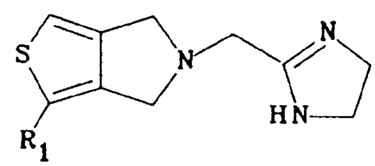

(I)

dans laquelle $R_1$ représente soit un groupe cyano, $C_{1-4}$ alcoxy, $C_{1-4}$ alkylthio, $C_{1-4}$ alkylsulfonyle, pyridyle, 3,4-diméthoxyphényle ou cyclopropyle éventuellement substitué par un ou deux groupes alkyle, soit un groupe COR où R est un groupe $C_{1-4}$ alkyle, phényle ou pipéridinyle, soit un groupe benzyle substitué par un ou plusieurs atomes d'halogène et/ou groupes $C_{1-4}$ alkyle linéaire ou ramifié, $C_{1-4}$ alcoxy linéaire ou ramifié ou $CO_2R'$ où R' est un groupe $C_{1-4}$ alkyle linéaire ou ramifié, soit un groupe naphtylméthyle.

Les composés de l'invention forment avec les acides pharmaceutiquement acceptables des sels qui font partie de l'invention.

Les composés de formule (I) sont préparés selon le procédé représenté dans l'annexe 1, qui consiste à traiter un composé de formule (III)

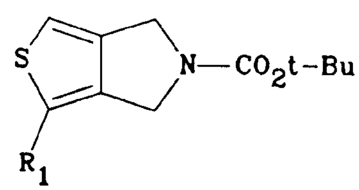

(III)

dans laquelle $R_1$ est défini comme dans la formule (I), par l'acide trifluoroacétique, à une température voisine de 0°C, puis à faire réagir le composé obtenu, de formule (II)

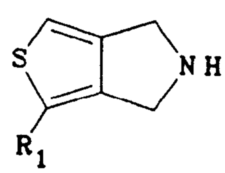

(II)

avec le 2-chlorométhyl-4,5-dihydro-1*H*-imidazole, dans un solvant tel que le diméthylformamide, en présence de N,N-diisopropyléthylamine.

Les composés de formule (III) sont eux-mêmes préparés à partir du 5,6-dihydro-4H-thiéno[3,4-*c*]pyrrole-5-carboxylate de 1,1-diméthyléthyle de formule (IV)

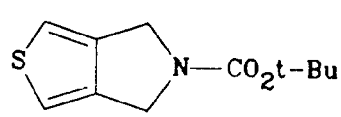

(IV)

selon des procédés qui dépendent de la nature du substituant $R_1$. Ces procédés sont représentés dans les annexes 2 et 3.

Les procédés représentés dans l'annexe 2 concernent la préparation des composés de formule (III) dans laquelle $R_1$ est un groupe $C_{1-4}$ alcoxy (composés VI), pyridyle ou 3,4-diméthoxyphényle (composés VII).

Selon ces procédés, on traite le composé de formule (IV) par une base forte, par exemple un composé de formule $R_3Li$ dans laquelle $R_3$ représente un groupe alkyle, en particulier n-butyle, dans un solvant tel que le tétrahydrofuranne, à une température voisine de -70°C, puis par un borate de trialkyle ou de triaryle, par exemple le borate de triméthyle, à la température ambiante et enfin par l'acide chlorhydrique, à une température voisine de -40°C, pour obtenir le 1-borono-5,6-dihydro-4H-thiéno[3,4-*c*]pyrrole-5-carboxylate de 1,1-diméthyléthyle de formule (V), qui est, soit traité par le peroxyde d'hydrogène à une température voisine de 45°C, puis par un sulfate de dialkyle de formule $(R_5O)_2SO_2$, dans laquelle $R_5$ représente un groupe $C_{1-4}$ alkyle linéaire ou ramifié, à la température ambiante, pour donner le composé de formule (VI) dans laquelle $R_5$ est défini comme précédemment,
soit traité par un halogénure de formule ArX, dans laquelle Ar représente un groupe pyridyle ou 3,4-diméthoxyphényle et X un atome d'halogène, en présence de palladium (0) et d'une base telle que l'hydroxyde de butyltriméthylammonium, dans un solvant tel que le méthanol, à la température de reflux, pour obtenir le composé de formule (VII) dans laquelle Ar est défini comme précédemment.

Les procédés représentés dans l'annexe 3 concernent la préparation des composés de formule (III) dans laquelle $R_1$ est un groupe cyano (composé IX), $C_{1-4}$ alkylthio (composés X), $C_{1-4}$ alkylsulfonyle (composés XI), un groupe RCO dans lequel R est un groupe $C_{1-4}$ alkyle ou phényle (composés XIII) ou un groupe cyclopropyle substitué ou non (composés XV).

Selon ces procédés, on traite le composé de formule (IV) par une base forte, par exemple un composé de formule $R_3Li$ dans laquelle $R_3$ représente un groupe alkyle, en particulier n-butyle, dans un solvant tel que le tétrahydrofuranne, à une température voisine de -70°C, puis on fait réagir le composé obtenu,
soit avec le diméthylformamide à la température ambiante, pour obtenir le composé de formule (VIII) que l'on traite par l'hydroxylamine puis par le carbonyldiimidazole, à la température ambiante pour obtenir le composé de formule (IX),
soit avec un composé de formule $R_5S_2$ dans laquelle $R_6$ représente un groupe $C_{1-4}$ alkyle, à la température ambiante pour obtenir le composé de formule (X), qui peut ensuite être oxydé par exemple au moyen de peroxymonosulfate de potassium, à la température ambiante pour donner le composé de formule (XI),
soit avec un composé de formule $R_7(R_8)C{=}O$ dans laquelle $R_7$ représente un atome d'hydrogène, un groupe alkyle ou aryle et $R_5$ un atome d'hydrogène ou un groupe alkyle, dans un solvant tel que le tétrahydrofuranne à la température ambiante pour obtenir le composé de formule (XII), dans laquelle $R_7$ et $R_5$ sont définis comme précédemment, qui, dans le cas où $R_8$ est un atome d'hydrogène, est traité par le dichromate de pyridinium en présence d'acide acétique, à la température ambiante pour donner le composé de formule (XIII) et, dans le cas où $R_8$ est un groupe alkyle, est traité par l'acide acétique en présence de chlorure de calcium pour donner le composé de formule (XIV) dans laquelle $R_7$ est défini comme précédemment et $R_9$ représente un atome d'hydrogène ou un groupe alkyle, qui est ensuite traité par le diiodométhane, pour donner le composé de formule (XV) dans laquelle $R_7$ et $R_9$ sont définis comme précédemment.

Les composés de formule (III) dans laquelle $R_1$ représente un groupe benzyle substitué, sont préparés selon un procédé analogue au procédé décrit dans la demande de brevet européen n° 93 402785.5 pour la préparation de composés de formule (III) comportant un groupe benzyle non substitué. Ce procédé consiste à traiter le composé de formule (IV) par un composé de formule $R_3Li$ dans laquelle $R_3$ est défini comme précédemment, dans un solvant tel que le tétrahydrofuranne, à une température voisine de -70°C, puis par un halogénure de benzyle convenablement substitué.
Les composés de formule (III) dans laquelle $R_1$ représente un groupe naphtylméthyle sont préparés par un procédé analogue, en remplaçant l'halogénure de benzyle par un halogénure de naphtylméthyle.

Le composé de formule (III) dans laquelle $R_1$ représente le groupe pipéridinylcarbonyle est préparé selon un procédé analogue au procédé décrit dans la demande de brevet français FR 93 07538 pour la préparation des composés de formule (III) comportant un groupe carbamoyle. Ce procédé consiste à traiter le composé de formule (III) dans laquelle $R_1$ représente un groupe carboxyle, par la pipéridine en présence d'un agent condensant.

La préparation du composé de formule (IV) est décrite dans la demande de brevet européen n° 93 402785.5.

Les exemples suivants illustrent l'invention.
Les exemples 2 à 11 concernent la préparation des composés de formule (III) selon les procédés représentés dans les annexes 2 et 3 et des composés de formule (II) correspondants.
Les analyses confirment la structure des composés.

Exemple 1 : Dichlorhydrate de 1-méthoxy-5-[(4,5-dihydro-1*H*-imidazol-2-yl)méthyl]-5,6-dihydro-4H-thiéno[3,4-*c*]pyrrole.

1.1. 1-Borono-5,6-dihydro-4H-thiéno[3,4-*c*]pyrrole-5-carboxylate de 1,1-diméthyléthyle.

A une solution de 2,29 ml (16,23 mmoles) de N,N-diisopropylamine dans 66 ml de tétrahydrofuranne sec, on ajoute à 0°C, 10,6 ml (17,03 mmoles) d'une solution 1,6 M de butyllithium dans de l'hexane. On agite le

mélange pendant 45 mn, puis on refroidit à -70°C et on ajoute une solution de 3g (13,31 mmoles) de 5,6-dihydro-4H-thiéno[3,4-*c*]pyrrole-5-carboxylate de 1,1-diméthyléthyle dans 26 ml de tétrahydrofuranne sec. On maintient le mélange à -70°C pendant 1 heure, puis on verse la solution obtenue sur une solution de 5 ml de borate de triméthyle dans 10 ml de tétrahydrofuranne, à la même température. On agite ensuite à température ambiante pendant une nuit, puis on refroidit le mélange réactionnel à -40°C et on ajoute 80 ml d'acide chlorhydrique 1N et 250 ml d'acétate d'éthyle. On décante la phase organique, on la lave avec 2 fois 60 ml de solution saturée de chlorure de sodium, puis on la sèche sur sulfate de sodium et on évapore le solvant. Après recristallisation du résidu dans de l'acétate d'éthyle, on obtient 2,4 g de produit.
Point de fusion : 160-162°C.

1.2. 1-Méthoxy-5,6-dihydro-4H-thiéno[3,4-*c*]pyrrole-5-carboxylate de 1,1-diméthyléthyle.

A une suspension de 4 g (14,9 mmoles) de 1-borono-5,6-dihydro-4H-thiéno[3,4-*c*]pyrrole-5-carboxylate de 1,1-diméthyléthyle dans 600 ml d'éther diéthylique, on ajoute 80 ml de peroxyde d'hydrogène à 30 %. On chauffe le mélange à 45°C pendant 20 mn, puis on décante la phase organique, on la lave avec 2 fois 100 ml d'une solution de carbonate de sodium à 5 % puis avec 200 ml d'une solution saturée de chlorure de sodium et on ajoute à 0°C, 600 ml de toluène, 49,94 g (147 mmoles) d'hydrosulfate de tétrabutylammonium, 67,8 ml (710 mmoles) de sulfate de diméthyle et 400 ml (800 mmoles) d'une solution d'hydroxyde de sodium 2N. On maintient 1,5 heure à température ambiante, puis on décante la phase organique, on la lave avec 2 fois 400 ml d'une solution d'hydrogènecarbonate de sodium à 7 %, on la sèche sur sulfate de sodium et on évapore le solvant. On chromatographie le résidu sur colonne de gel de silice, avec un mélange 1/9 d'acétate d'éthyle et d'hexane. On obtient 0,95 g de produit. Point de fusion : 62,8-64,7°C.

1.3. Dichlorhydrate de l-méthoxy-5-[(4,5-dihydro-1*H*-imidazol-2-yl)méthyl]-5,6-dihydro-4H-thiéno[3,4-*c*]pyrrole.

On agite pendant 10 mn, à 0°C, un mélange de 0,95 g (3,72 mmoles) de 1-méthoxy-5,6-dihydro-4H-thiéno[3,4-*c*]pyrrole-5-carboxylate de 1,1-diméthyléthyle et de 8,5 ml d'acide trifluoroacétique, puis on évapore le solvant et on dissout le résidu dans 21 ml de diméthylformamide. On ajoute 1,27 ml (7,5 mmoles) de N,N-diisopropyléthylamine puis une solution de 0,577 g (3,72 mmoles) de chlorhydrate de 2-chlorométhyl-4,5-dihydro-1*H*-imidazole et de 0,53 ml (3,1 mmoles) de N,N-diisopropyléthylamine dans 21 ml de diméthylformamide. On laisse le mélange une nuit à température ambiante, puis on évapore le solvant et on ajoute 35 ml d'eau et 35 ml d'une solution d'hydrogènecarbonate de sodium à 7 %. On extrait avec 6 fois 60 ml de dichlorométhane, on sèche les phases organiques sur sulfate de sodium et on évapore le solvant. On chromatographie le résidu sur colonne de gel de silice avec un mélange 1/9 de méthanol et de dichlorométhane. On obtient 0,49 g d'un produit huileux qui est transformé en chlorhydrate par addition d'une solution saturée d'acide chlorhydrique dans de l'isopropanol. On obtient 0,31 g de produit.
Point de fusion : 243-245°C (avec décomposition).

<u>Exemple 2</u> : Trifluoroacétate de 1-(3,4-diméthoxyphényl)-5,6-dihydro-4H-thiéno[3,4-*c*]pyrrole.

2.1. 1-(3,4-Diméthoxyphényl)-5,6-dihydro-4H-thiéno[3,4-*c*]pyrrole-5-carboxylate de 1,1-diméthyléthyle.

Dans un ballon de 250 ml, on introduit 3 g (11,14 mmoles) de 1-borono-5,6-dihydro-4H-thiéno[3,4-*c*]pyrrole-5-carboxylate de 1,1-diméthyléthyle, 1,53 ml (12,03 mmoles) de 1-bromo-3,4-diméthoxybenzène, 0,9 g (0,78 mmoles) de tétrakistriphénylphosphine palladium (0), 10 ml (22,8 mmoles) d'une solution d'hydroxyde de butyltriméthylammonium à 40 % dans du méthanol et 60 ml de méthanol. On chauffe le mélange au reflux pendant 6 heures et on évapore le solvant. On reprend ensuite le résidu avec 150 ml de dichlorométhane, on lave avec 2 fois 100 ml d'une solution saturée d'hydrogènecarbonate de sodium, on sèche sur sulfate de sodium et on évapore le solvant. On chromatographie le résidu sur colonne de gel de silice avec un mélange 1/5 d'acétate d'éthyle et d'hexane. On obtient 1,32 g de produit.
Point de fusion : 130-132°C.

2.2. Trifluoroacétate de 1-(3,4-diméthoxyphényl)-5,6-dihydro-4H-thiéno[3,4-*c*]pyrrole.

On agite pendant 10 mn, à 0°C, une solution de 1,32 g (3,65 mmoles) de 1-(3,4-diméthoxyphényl)-5,6-dihydro-4H-thiéno[3,4-*c*]pyrrole-5-carboxylate de 1,1-diméthyléthyle dans 12 ml d'acide trifluoroacétique. Après évaporation du solvant, on obtient 1,37 g d'un produit huileux.

Exemple 3 : Trifluoroacétate de 1-pyrid-2-yl-5,6-dihydro-4H-thiéno[3,4-*c*]pyrrole.

Ce composé est obtenu par un procédé analogue à celui de l'exemple 2, à partir de la 2-bromopyridine.

Exemple 4 : Trifluoroacétate de 1-cyano-5,6-dihydro-4H-thiéno[3,4-*c*]pyrrole.

4.1. 1-Formyl-5,6-dihydro-4H-thiéno[3,4-*c*]pyrrole-5-carboxylate de 1,1-diméthyléthyle.

A une solution de 3,2 ml (22,7 mmoles) de diisopropylamine dans 84 ml de tétrahydrofuranne sec, on ajoute, à 0°C, 15,55 ml (24,88 mmoles) d'une solution 1,6 M de butyllithium dans de l'hexane. Après 30 mn, on refroidit à -70 °C et on ajoute une solution de 5 g (22,2 mmoles) de 5,6-dihydro-4H-thiéno[3,4-*c*]pyrrole-5-carboxylate de 1,1-diméthyléthyle dans 34 ml de tétrahydrofuranne, refroidie à -70°C. On agite ensuite pendant 30 mn à 0°C, puis on ajoute à -70°C, 10 ml de diméthylformamide. On agite à température ambiante pendant 30 mn, puis on ajoute 34 ml d'une solution saturée de chlorure d'ammonium. On décante la phase organique, on extrait la phase aqueuse avec 2 fois 50 ml d'acétate d'éthyle, puis on réunit les phases organiques, on les lave avec une solution saturée de chlorure de sodium et on évapore le solvant. On chromatographie le résidu sur colonne de gel de silice avec un mélange 1/10 d'acétate d'éthyle et d'hexane. On obtient 4,6 g de produit.
Point de fusion : 84,3-86,6°C.

4.2. 1-[(Hydroxyimino)méthyl]-5,6-dihydro-4H-thiéno[3,4-*c*]pyrrole-5-carboxylate de 1,1-diméthyléthyle.

Dans un ballon de 500 ml, on introduit 3,59 g (14,17 mmoles) de 1-formyl-5,6-dihydro-4H-thiéno[3,4-*c*]pyrrole-5-carboxylate de 1,1-diméthyléthyle, 11,56 g (85 mmoles) de trihydrate d'acétate de sodium, 4,91 g (70,85 mmoles) de chlorhydrate d'hydroxylamine et 180 ml de méthanol. On agite le mélange à température ambiante pendant 3 heures, puis on évapore le solvant et on ajoute 200 ml d'une solution saturée de chlorure de sodium et 200 ml de dichlorométhane. On décante la phase organique et on extrait la phase aqueuse avec 2 fois 150 ml de dichlorométhane. On réunit les phases organiques, on les sèche sur sulfate de sodium et on évapore le solvant. On obtient 1,76 g de produit.
Point de fusion : 193,8-194,9°C.

4.3. 1-Cyano-5,6-dihydro-4H-thiéno[3,4-*c*]pyrrole-5-carboxylate de 1,1-diméthyléthyle.

A une suspension de 1,73 g (6,44 mmoles) de 1-[(hydroxy-imino)méthyl]-5,6-dihydro-4H-thiéno[3,4-c]pyrrole-5-carboxylate de 1,1-diméthyléthyle dans 41 ml de chloroforme, on ajoute à 0°C, 3,12 g (19,34 mmoles) de carbonyl-diimidazole. On agite le mélange à température ambiante pendant 24 heures, puis on le verse sur un mélange de 200 ml d'acide chlorhydrique 1N et 200 ml de dichlorométhane, refroidi à 0°C. On décante la phase organique, on la sèche sur sulfate de sodium puis on évapore le solvant. On obtient 1,58 g de produit.
Point de fusion : 106,2-107,3°C.

4.4. Trifluoroacétate de 1-cyano-5,6-dihydro-4H-thiéno[3,4-*c*]pyrrole.

On agite pendant 10 mn, à 0°C, une solution de 1,3 g (5,19 mmoles) de 1-cyano-5,6-dihydro-4H-thiéno[3,4-*c*]pyrrole-5-carboxylate de 1,1-diméthyléthyle dans 11 ml d'acide trifluoroacétique, puis on évapore le solvant. On obtient 1,37 g de produit.
Point de fusion : 87,5-89,7°C.

Exemple 5 : Trifluoroacétate de 1-(1-méthylcyclopropyl)-5,6-dihydro-4H-thiéno[3,4-*c*]pyrrole.

5.1. 1-(1-Méthylcyclopropyl)-5,6-dihydro-4H-thiéno[3,4-*c*]pyrrole-5-carboxylate de 1,1-diméthyléthyle.

A une suspension de 1,45 g (5,46 mmoles) de 1-(1-propèn-2-yl)-5,6-dihydro-4H-thiéno[3,4-*c*]pyrrole-5-carboxylate de 1,1-diméthyléthyle dans 80 ml de n-hexane, refroidie à -23°C, on ajoute 7,05 ml (7,05 mmoles) d'une solution 1M de diéthylzinc dans de l'hexane puis 21,96 g (82 mmoles) de diiodométhane. On agite à -23°C pendant 6 heures puis à 4°C pendant 19 heures, puis on ajoute 200 ml d'éther diéthylique et 50 ml d'une solution saturée de chlorure d'ammonium. On décante la phase organique, on la sèche sur sulfate de sodium et on évapore le solvant. On chromatographie le résidue sur colonne de gel de silice avec un mélange 1/15 d'acétate d'éthyle et d'hexane. On obtient 0,46 g d'un produit huileux.

5.2. Trifluoroacétate de 1-(1-méthylcyclopropyl)-5,6-dihydro-4H-thiéno[3,4-*c*]pyrrole.

On agite pendant 10 mn, à 0°C, une solution de 0,905 g (3,24 mmoles) de 1-(1-méthylcyclopropyl)-5,6-dihydro-4H-thiéno[3,4-*c*]pyrrole-5-carboxylate de 1,1-diméthyléthyle dans 6 ml d'acide trifluoroacétique, puis on évapore le solvant. On obtient 0,95 g d'un produit huileux.

<u>Exemple 6</u> : Trifluoroacétate de 1-méthylthio-5,6-dihydro-4H-thiéno[3,4-*c*]pyrrole.

6.1. 1-Méthylthio-5,6-dihydro-4H-thiéno[3,4-*c*]pyrrole-5-carboxylate de 1,1-diméthyléthyle.

A une solution de 6,5 ml (46,3 mmoles) de N,N-diisopropylamine dans 130 ml de tétrafuranne sec, on ajoute à 0°C, 30 ml (48 mmoles) d'une solution 1,6 M de butyllithium dans de l'hexane. Après 30 mn, on refroidit le mélange à -70°C et on ajoute une solution de 8,7 g (38,59 mmoles) de 5,6-dihydro-4H-thiéno[3,4-*c*]pyrrole-5-carboxylate de 1,1-diméthyléthyle dans 30 ml de tétrahydrofuranne sec. Après 1 heure à -70°C, on ajoute 4,86 ml (54 mmoles) de disulfure de diméthyle, puis on agite à température ambiante pendant 1 heure. On verse ensuite le mélange réactionnel dans 100 ml d'une solution saturée de chlorure d'ammonium puis on extrait avec 2 fois 100 ml de dichlorométhane. On sèche les phases organiques sur sulfate de sodium et on évapore le solvant. Après cristallisation dans l'hexane, on obtient 10 g de produit.
Point de fusion : 51-53°C.

6.2. Trifluoroacétate de 1-méthylthio-5,6-dihydro-4H-thiéno[3,4-*c*]pyrrole.

On agite pendant 1 heure, à une température voisine de 0°C, une solution de 4,88 g (17,9 mmoles) de 1-méthylthio-5,6-dihydro-4H-thiéno[3,4-*c*]pyrrole-5-carboxylate de 1,1-diméthyléthyle dans 10 ml d'acide trifluoroacétique, puis on évapore le solvant. On chromatographie le résidu sur colonne de gel de silice avec un mélange 1/9 de méthanol et de dichlorométhane. On dissout le produit obtenu dans 1 ml d'acide trifluoroacétique puis on évapore le solvant. On obtient 5 g d'un produit huileux.

<u>Exemple 7</u> : Trifluoroacétate de 1-méthylsulfonyl-5,6-dihydro-4H-thiéno[3,4-*c*]pyrrole.

7.1. 1-Méthylsulfonyl-5,6-dihydro-4H-thiéno[3,4-*c*]pyrrole-5-carboxylate de 1,1-diméthyléthyle.

A une solution de 2,1 g (7,8 mmoles) de 1-méthylthio-5,6-dihydro-4H-thiéno[3,4-*c*]pyrrole-5-carboxylate de 1,1-diméthyléthyle dans 50 ml d'éthanol, on ajoute à une température voisine de -20°C, une solution de 9,5 g (15,5 mmoles) de peroxymonosulfate de potassium dans 50 ml d'eau. On agite pendant 3 heures à température ambiante puis on filtre et on élimine l'éthanol. On extrait la phase aqueuse avec 4 fois 50 ml d'acétate d'éthyle, puis on sèche les phases organiques sur sulfate de sodium et on évapore le solvant. On chromatographie le résidu sur colonne de gel de silice avec un mélange 3/7 d'acétate d'éthyle et d'hexane. On obtient 1,1 g de produit.
Point de fusion : 122-125°C.

7.2. Trifluoroacétate de 1-méthylsulfonyl-5,6-dihydro-4H-thiéno[3,4-*c*]pyrrole.

On agite pendant 1 heure, à une température voisine de 0°C, une solution de 1 g (3,3 mmoles) de 1-méthylsulfonyl-5,6-dihydro-4H-thiéno[3,4-*c*]pyrrole-5-carboxylate de 1,1-diméthyléthyle dans 8 ml d'acide trifluoroacétique, puis on évapore le solvant. On ajoute 10 ml d'éther diéthylique et on filtre le précipité formé. On obtient 1 g de produit.
Point de fusion : 172-176°C.

<u>Exemple 8</u> : Trifluoroacétate de 1-(pipéridin-1-ylcarbonyl)-5,6-dihydro-4H-thiéno[3,4-*c*]pyrrole.

8.1. 1-(Pipéridin-1-ylcarbonyl)-5,6-dihydro-4H-thiéno[3,4-*c*]pyrrole-5-carboxylate de 1,1-diméthyléthyle.

A une solution de 1,95 g (7,2 mmoles) de 1-carboxy-5,6-dihydro-4H-thiéno[3,4-*c*]pyrrole-5-carboxylate de 1,1-diméthyléthyle dans 50 ml de tétrahydrofuranne sec, on ajoute une solution de 1,29 g (7,9 mmoles) de carbonyldiimidazole dans 12 ml de tétrahydrofuranne. Après 1 heure à température ambiante, on ajoute 5 ml de pipéridine et on agite 30 mn à température ambiante. On évapore le solvant, on ajoute 40 ml d'eau et on extrait avec 2 fois 50 ml d'acétate d'éthyle. on sèche les phases organiques sur sulfate de sodium et on évapore

le solvant. On obtient 1,89 g d'un produit huileux.

8.2. Trifluoroacétate de 1-(pipéridin-1-ylcarbonyl)-5,6-dihydro-4H-thiéno[3,4-*c*]pyrrole.

On agite pendant 30 mn, à une température voisine de 0°C, une solution de 1,86 g (5,5 mmoles) de 1-(pipéridin-1-ylcarbonyl)-5,6-dihydro-4H-thiéno[3,4 *c*]pyrrole-5-carboxylate de 1,1-diméthyléthyle dans 10 ml d'acide trifluoroacétique, puis on évapore le solvant. On obtient 1,96 g d'un produit huileux.

Exemple 9 : Trifluoroacétate de 1-acétyl-5,6-dihydro-4H-thiéno[3,4-*c*]pyrrole.

9.1. 1-(1-Hydroxy-1-éthyl)-5,6-dihydro-4H-thiéno[3,4-*c*]pyrrole-5-carboxylate de 1,1-diméthyléthyle.

A une solution de 0,72 g (7,2 mmoles) de N,N-diisopropylamine dans 10 ml de tétrahydrofuranne sec, on ajoute à 0°C, 4,5 ml (7,2 mmoles) d'une solution 1,6 M de butyllithium dans de l'hexane. Après 30 mn, on refroidit à -40°C et on ajoute une solution de 1,35 g (6 mmoles) de 5,6-dihydro-4H-thiéno[3,4-*c*]pyrrole-5-carboxylate de 1,1-diméthyléthyle dans 10 ml de tétrahydrofuranne. Après 1 heure à -40°C, on refroidit à -70°C et on ajoute 1 ml (18 mmoles) d'acétaldéhyde puis on agite à température ambiante pendant 2 heures. On verse ensuite le mélange réactionnel dans 30 ml d'une solution saturée de chlorure d'ammonium puis on extrait avec 2 fois 50 ml d'acétate d'éthyle. On sèche les phases organiques sur sulfate de sodium puis on évapore le solvant. On chromatographie le résidu sur colonne de gel de silice avec un mélange 1/4 d'acétate d'éthyle et d'hexane. On obtient 1,19 g d'un produit huileux.

9.2. 1-Acétyl-5,6-dihydro-4H-thiéno[3,4-*c*]pyrrole-5-carboxylate de 1,1-diméthyléthyle.

A une solution de 5,39 g (20 mmoles) de 1-(1-hydroxy-1-éthyl)-5,6-dihydro-4H-thiéno[3,4-*c*]pyrrole-5-carboxylate de 1,1-diméthyléthyle dans 100 ml de dichlorométhane, on ajoute 1,14 ml (20 mmoles) d'acide acétique puis 5,64 g (15 mmoles) de dichromate de pyridinium. On agite le mélange pendant 16 heures à la température ambiante, puis on ajoute 4 g de célite, on filtre et on évapore le solvant.
On chromatographie le résidu sur colonne de gel de silice avec un mélange 35/65 d'acétate d'éthyle et d'hexane. On obtient 2,16 g de produit.
Point de fusion : 119-121°C.

9.3. Trifluoroacétate de 1-acétyl-5,6-dihydro-4H-thiéno[3,4-*c*]pyrrole.

On agite pendant 30 mn, à une température voisine de 0°C, une solution de 1,87 g (7 mmoles) de 1-acétyl-5,6-dihydro-4H-thiéno[3,4-*c*]pyrrole-5-carboxylate de 1,1-diméthyléthyle dans 8 ml d'acide trifluoroacétique. On évapore ensuite le solvant, on ajoute 20 ml d'éther diéthylique et on filtre. On obtient 1,76 g de produit.
Point de fusion : 136-138°C.

Exemple 10 : Trifluoroacétate de 1-benzoyl-5,6-dihydro-4H-thiéno[3,4-*c*]pyrrole.

Ce composé est obtenu par un procédé analogue à celui de l'exemple 9, en remplaçant l'acétaldéhyde par le benzaldéhyde.

Exemple 11 : Trifluoroacétate de 1-propylcarbonyl-5,6-dihydro-4H-thiéno[3,4-*c*]pyrrole.

Ce composé est obtenu par un procédé analogue à celui de l'exemple 9, en remplaçant l'acétaldéhyde par le butyraldéhyde.

Les composés de l'invention sont rassemblés dans le tableau I avec leurs caractéristiques physiques.

Tableau I

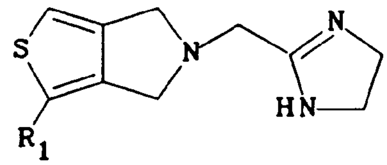

(I)

| Composé | $R_1$ | sel | F (°C) |
|---|---|---|---|
| 1 | H3CO- | dichlorhydrate | 202-204 (d) |
| 2 | 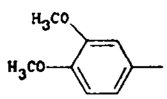 | diméthanesulfonate | 194-195 (d) |
| 3 | 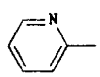 | triméthanesulfonate | 161-162 (d) |
| 4 | NC- | diméthanesulfonate | 205-206 |
| 5 | 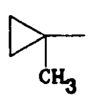 | dichlorhydrate | 224-226 (d) |
| 6 | H3CS- | dichlorhydrate | 227-229 (d) |
| 7 | H3CSO2- | diméthanesulfonate | 192-194 (d) |
| 8 | 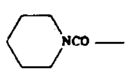 | dichlorhydrate | 206-208 (d) |
| 9 | H3CCO- | dichlorhydrate | > 270 (d) |
| 10 | 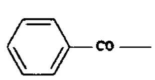 | dichlorhydrate | > 270 (d) |
| 11 | $H_3C(CH_2)_2CO$- | dichlorhydrate | 198-200 (d) |
| 12 | 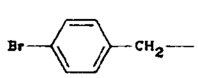 | dichlorhydrate | 246-248 |

| Composé | $R_1$ | sel | F (°C) |
|---|---|---|---|
| 13 | 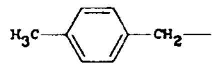 | dichlorhydrate | 235-237 |
| 14 | 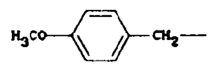 | dichlorhydrate | 226-227 (d) |
| 15 | 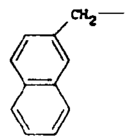 | dichlorhydrate | 232-233 (d) |

d = décomposition

Les composés de l'invention présentent une activité pharmacologique $\alpha_2$-antagoniste et ont été testés dans différents essais biologiques.

1. Antagonisme des effets de la clonidine sur le vas deferens de rat.

Cette détermination a eu lieu sur le vas deferens du rat stimulé à une fréquence de 0,1 Hz en présence de 30 nanomoles de prazosine et de une micromole de cocaïne selon la méthode décrite par G.M. Drew dans European Journal of Pharmacology, 42, 123-130, (1977).
Les $pA_2$ des composés de l'invention sont compris entre 6,5 et 9,4.

2. Antagonisme de la liaison de la $^3$H-clonidine sur les récepteurs $\alpha_2$-adrénergiques.

Le test est réalisé sur une préparation de membranes de cerveau de rat, selon la méthode décrite par D.A. Greenberg et al. dans Life Sci. 19, 69, (1976).
Après 30 mn d'incubation en présence de clonidine tritiée (0,05 à 7 nmole/l), on filtre et on procède au comptage de la radioactivité du résidu selon la méthode de P.B.M.W.M. Timmermans et al., décrite dans European Journal of Pharmacology, 70, 7, (1981).
Les concentrations inhibitrices 50 des composés de l'invention sont comprises entre 0,02 et 3,02 µmole/l.

Les résultats des essais biologiques montrent que les composés de l'invention présentent in vitro des propriétés antagonistes vis-à-vis des récepteurs adrénergiques de type $\alpha_2$. Les composés de l'invention peuvent être utilisés, compte tenu de leurs propriétés pharmacologiques, pour le traitement du diabète, de l'obésité, de l'hypotension, de l'iléum paralytique post-opératoire et/ou de l'asthme.

Les composés de l'invention présentent également une activité $\alpha_1$-agoniste, mise en évidence par des essais biologiques sur l'artère pulmonaire isolée de lapin.

Ces essais ont été réalisés dans les conditions suivantes: des lapins (Fauve de Bourgogne), pesant 2 à 3 kg, ont été assommés et saignés, puis leurs artères pulmonaires ont été prélevées, disséquées et découpées en bandelettes d'environ 1,2 à 2 mm de large et 20 mm de long.
Ces bandelettes de tissu vasculaire ont été plongées dans une solution physiologique (composition, exprimée en mmol./l : chlorure de sodium 137 ; chlorure de potassium 2,7 ; chlorure de calcium 1,8 ; dihydrogénophosphate de sodium 0,4 ; hydrogénocarbonate de sodium 11,9 ; hexahydrate de chlorure de magnésium 1,1 ; dextrose 5,9 ; sel disodique de l'acide éthylènediaminetétraacétique 0,027 et acide ascorbique 0,057), oxygénée avec un mélange de 95% d'oxygène et de 5% de gaz carbonique et maintenue à une température de 37°C.
Elles ont ensuite été soumises, pendant 4 h, à une traction de 4 g, réduite à 2 g juste avant le début de l'expérience. On a alors contracté le tissu avec le composé à étudier et on a enregistré la tension résultante à l'aide d'un polygraphe Grass, modèle 7D et un transducteur de force. On a tracé deux courbes concentration-effet, avec des concentrations cumulatives de composé (de 100 nmol./l à 3 mmol./l) puis on a ajouté dans le bain, un $\alpha_1$-antagoniste, l'alfuzosine, à la concentration de 1 µmol./l, laissé en contact avec le tissu pendant

30 mn. Une autre courbe concentration-effet a alors été tracée et comparée avec la seconde courbe de contrôle.

L'effet $\alpha_1$-agoniste est mesuré par la concentration provoquant une contraction égale à 50 % de l'effet maximal.

Pour les composés de l'invention, cette concentration varie entre 1,3 et 2,6 µmol./l.

Ces résultats montrent que les composés de l'invention présentent in vitro des propriétés agonistes vis-à-vis des récepteurs adrénergiques de type $\alpha_1$. Les composés de l'invention peuvent donc être utilisés dans le traitement de l'incontinence urinaire.

Les composés de l'invention peuvent être présentés sous toute forme appropriée, en association avec tout excipient approprié, pour l'administration par voie orale ou parentérale ; par exemple sous la forme de comprimés, de dragées, de capsules, de solutions, etc..;

La posologie quotidienne peut aller de 0,1 à 20 mg/kg par voie orale.

## Annexe 1

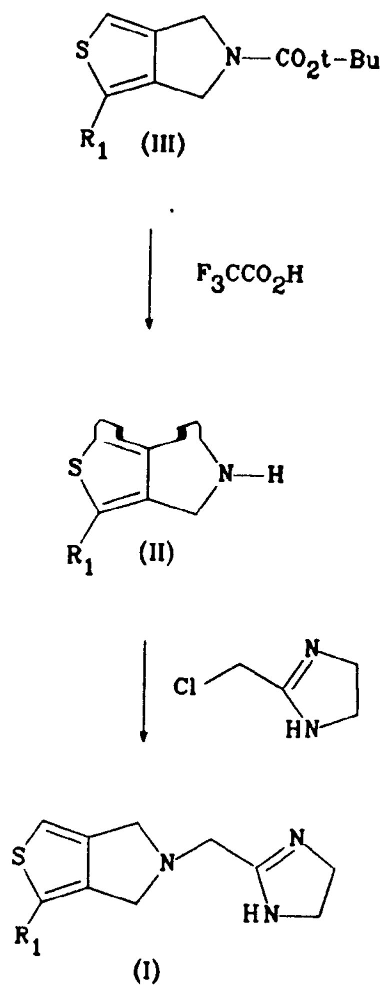


(III)

(II)

(I)

## Annexe 2

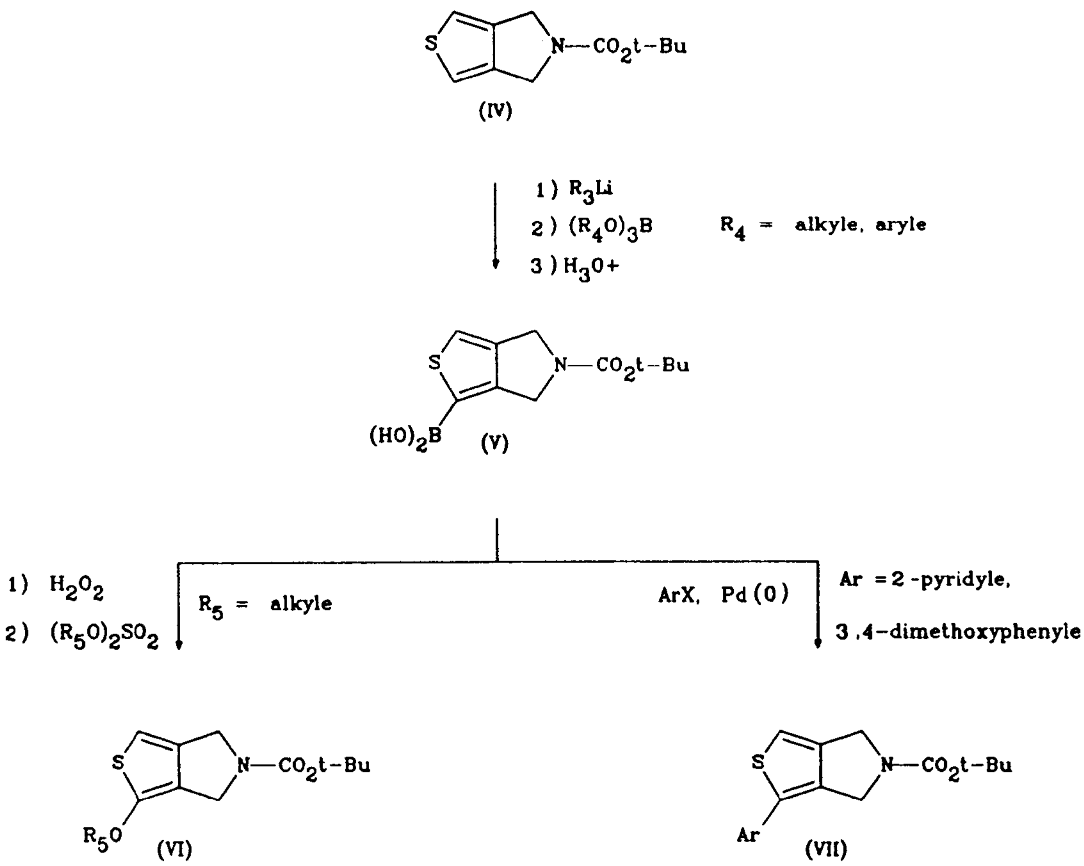
(IV)
1) $R_3Li$
2) $(R_4O)_3B$
3) $H_3O+$
$R_4$ = alkyle, aryle
(V)
1) $H_2O_2$
2) $(R_5O)_2SO_2$
$R_5$ = alkyle
ArX, Pd (0)
Ar = 2 -pyridyle,
3 ,4-dimethoxyphenyle
(VI)
(VII)

## Annexe 3

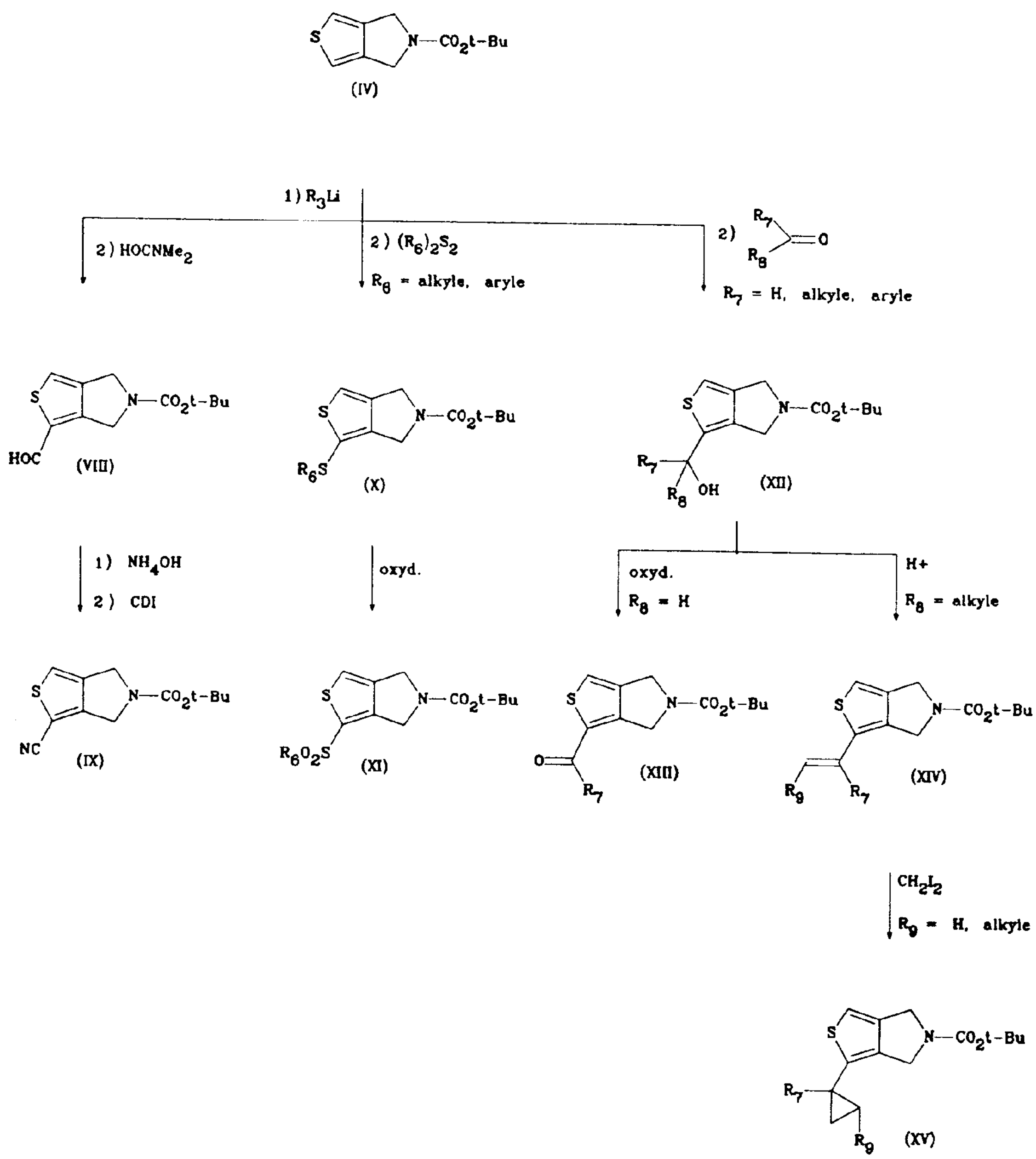


## Revendications

1. Dérivés de 5,6-dihydro-4*H*-thiéno[3,4-*c*]pyrrole de formule générale (I)

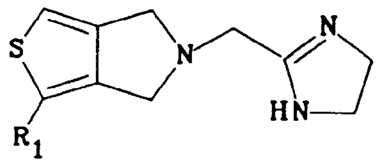

(I)

dans laquelle $R_1$ représente soit un groupe cyano, $C_{1-4}$ alcoxy, $C_{1-4}$ alkylthio, $C_{1-4}$ alkylsulfonyle, pyridyle, 3,4-diméthoxyphényle ou cyclopropyle éventuellement substitué par un ou deux groupes alkyle, soit un groupe COR où R est un groupe $C_{1-4}$ alkyle, phényle ou pipéridinyle, soit un groupe benzyle substitué par un ou plusieurs atomes d'halogène et/ou groupes $C_{1-4}$ alkyle linéaire ou ramifié, $C_{1-4}$ alcoxy linéaire ou ramifié ou $CO_2R'$ où R' est un groupe $C_{1-4}$ alkyle linéaire ou ramifié, soit un groupe naphtylméthyle, ainsi que leurs sels d'addition à des acides pharmaceutiquement acceptables.

2. Le dichlorhydrate de 1-méthoxy-5-[(4,5-dihydro-1*H*-imidazol-2-yl)méthyl]-5,6-dihydro-4H-thiéno[3,4-*c*]pyrrole.

3. Le dichlorhydrate de 1-méthylthio-5-[(4,5-dihydro-1*H*-imidazol-2-yl)méthyl]-5,6-dihydro-4H-thiéno[3,4-*c*]pyrrole.

4. Procédé de préparation des composés de formule (I) selon la revendication 1, caractérisé en ce que l'on fait réagir un composé de formule (II)

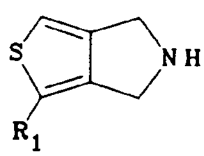

(II)

dans laquelle R1 est défini comme dans la revendication 1, avec le 2-chlorométhyl-4,5-dihydro-1H-imidazole, dans un solvant tel que le diméthylformamide, en présence de N,N-diisopropyléthylamine.

5. Médicament caractérisé en ce qu'il consiste en un composé de formule (I) selon la revendication 1.

6. Composition pharmaceutique caractérisée en ce qu'elle contient un composé de formule (I) selon la revendication 1 en association avec tout excipient approprié.

7. En tant qu'intermédiaire de synthèse pour la préparation des composés de formule (I) dans laquelle $R_1$ représente un groupe $C_{1-4}$ alcoxy, pyridyle ou 3,4-diméthoxyphényle, le 1-borono-5,6-dihydro-4H-thiéno[3,4-*c*]pyrrole-5-carboxylate de 1,1-diméthyléthyle.

Office européen des brevets

# RAPPORT DE RECHERCHE EUROPEENNE

Numero de la demande
EP 95 40 1015

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 238 753 (BEECHAM GROUP)<br>* revendications 1,10,13 * | 1,6 | C07D495/04<br>C07F5/02<br>A61K31/415<br>//(C07D495/04, 333:00,209:00) |
| A | CHEMICAL ABSTRACTS, vol. 67, no. 7, 1967, Columbus, Ohio, US;<br>abstract no. 32526a,<br>K. YU. NOVITSKII ET AL.: 'Furan series. XL. Reaction of 3,4-bis(chloromethyl)furan with primary amines'<br>page 3070 ;<br>* abrégé *<br>& KHIM. GETEROTSIKL. SOEDIN, no.6, 1966<br>page 818-821 | 1 | |
| A | JOURNAL OF ORGANIC CHEMISTRY, vol.34, no.2, Février 1969, EASTON, US;<br>pages 333 - 340<br>D.J. ZWANENBURG: 'Steric inhibition of intramolecular cyclizations by ortho substituents. The synthesis of 1H,3H-thieno[3,4-c]thiophene, its 2,2-dioxide, and 5-ethyl-5,6-dihydro-4H-thieno[3,4-c]pyrrole'<br>* page 336, composé 28 * | 1 | **DOMAINES TECHNIQUES RECHERCHES (Int.Cl.6)**<br>C07D<br>A61K<br>C07F |
| A | WO-A-93 03714 (UPJOHN CO.)<br>* revendication 1; exemples 95,104 * | 1,6 | |
| P,A | EP-A-0 599 697 (SYNTHELABO)<br>* revendications 1,10 * | 1,6 | |

Le présent rapport a été établi pour toutes les revendications

| Lieu de la recherche | Date d'achèvement de la recherche | Examinateur |
|---|---|---|
| LA HAYE | 21 Juin 1995 | Voyiazoglou, D |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)